(19) Europäisches Patentamt
European Patent Office
Office européen des brevets

(11) Publication number: 0 161 827
A1

(12) # EUROPEAN PATENT APPLICATION

(21) Application number: 85302767.0

(22) Date of filing: 19.04.85

(51) Int. Cl.4: C 07 C 69/757, C 07 C 67/02

(30) Priority: 25.04.84 GB 8410590

(43) Date of publication of application: 21.11.85
Bulletin 85/47

(84) Designated Contracting States: CH DE FR GB IT LI NL

(71) Applicant: INDIAN EXPLOSIVES LIMITED, ICI
House 34 Chowringhee Road, Calcutta-700 071 West
Bengal (IN)

(72) Inventor: Mandal, Arun Kanti Alchemie Research Centre,
Private Limited CAFI Site P.O. Box 155,
Thane 400 601 Maharashtra (IN)
Inventor: Soni, Nandkumar Ramgopal Alchemie
Research Centre, Private Limited CAFI Site P.O.
Box 155, Thane 400 601 Maharashtra (IN)
Inventor: Ratnam, K. Rakesh Alchemie Research Centre,
Private Limited CAFI Site P.O. Box 155,
Thane 400 601 Maharashtra (IN)

(74) Representative: Collier, Jeremy Austin Grey et al,
J.A.Kemp & Co. 14, South Square Gray's Inn, London
WC1R 5EU (GB)

(54) **Novel intermediate for the preparation of cyclopropane carboxylate esters and its preparation and use.**

(57) A novel compound, (1R, Cis)-3'-phenoxybenzyl-2,2-dimethyl-3-hydroxymethyl cyclopropane carboxylate:

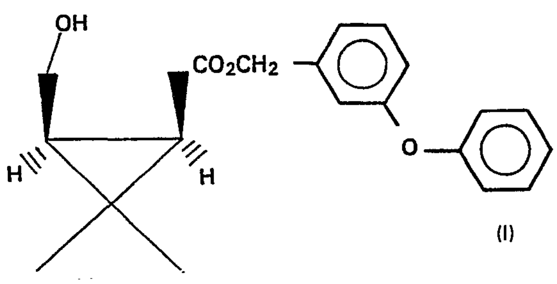

(I)

is a useful starting material for the production of optically active pyrethroid insecticidal compounds possessing a (1R, Cis) configuration. It may be made by hydrolysing the γ-lactone of (1R, Cis)-2,2-dimethyl-3-hydroxymethyl cyclopropane-1-carboxylic acid and alkylating the resulting intermediate with 3'-phenoxy-benzyl halide.

EP 0 161 827 A1

# NOVEL INTERMEDIATE FOR THE PREPARATION OF CYCLOPROPANE CARBOXYLATE ESTERS AND ITS PREPARATION AND USE.

This invention relates to cyclopropane carboxylate esters having insecticidal activity and to their preparation.

The cyclopropane carboxylate esters generally known as "Pyrethroids" are insecticidally active. One class of these Pyrethroid compounds may be represented by the general formulae:

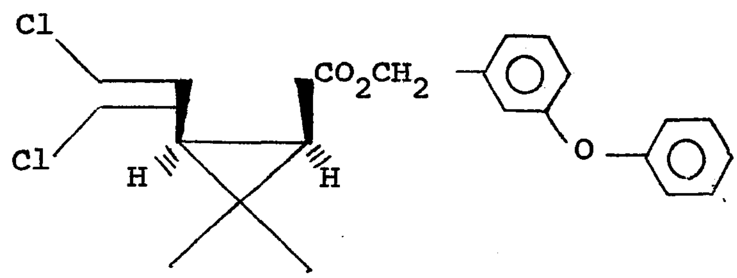

(II)

and

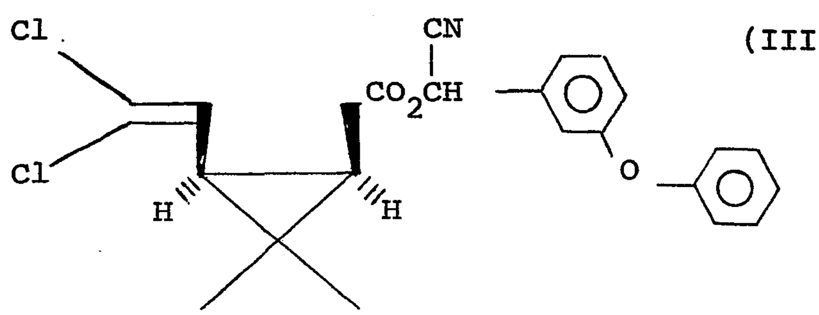

(III)

It has been established that the stereoisomeric form of the acid portion of the esters (II) and (III) should have the (1R, Cis) configuration for maximum insecticidal activity.

The present invention provides the novel compound (1R, Cis)-3'-phenoxybenzyl-2,2-dimethyl-3-hydroxymethyl cyclopropane carboxylate of the formula:

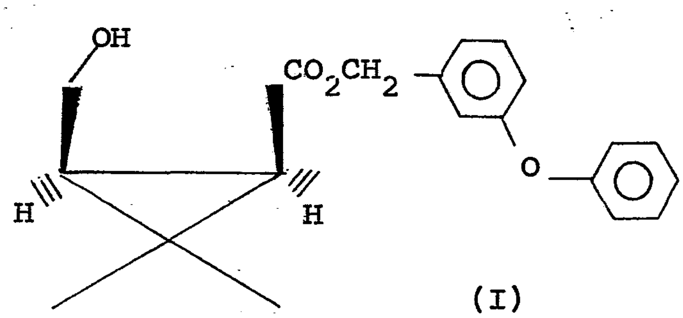

(I)

which is a useful intermediate for the preparation of pyrethroid insecticides of formula II.

The invention also provides a process for the manufacture of the said (1R, Cis)-cyclopropane-carboxylate ester of formula (I) from the γ-lactone of (1R, Cis)-2,2-dimethyl-3-hydroxymethyl cyclopropane-1-carboxylic acid of the formula:

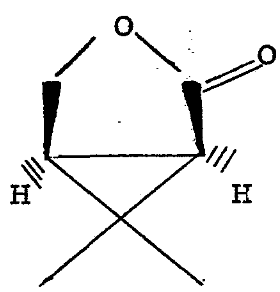

(IV)

which comprises, in a first step, hydrolysing the γ -lactone of (1R, Cis)-2,2-dimethyl-3-hydroxymethyl cyclopropane-1-carboxylic acid of formula (IV), preferably with a base, and, in a second step, alkylating the intermediate thus formed with a 3'-phenoxybenzyl halide of formula (V)

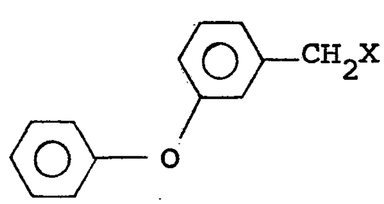

(V)

wherein X represents a halide, preferably chlorine, bromine or iodine.

The starting material of formula (IV) may be obtained by the process described in European Patent Application No. 84300215.5 filed on January 13th 1984.

Preferably, the step of hydrolysis of the compound of formula IV is carried out in the presence of a solvent. In this step of the process, the base which can be used may be an alkali metal hydroxide, alkaline earth metal hydroxide, or quaternary ammonium hydroxide, and is preferably sodium hydroxide, potassium hydroxide or tetra-n-butyl-ammonium hydroxide. Suitable solvents are alkanols having 1-4 carbon atoms, preferably ethanol, dichloromethane, and tetrahydrofuran. The reaction can be carried out at a mild temperature, e.g. between 0° to 100°C, but preferably at ambient temperature.

In the second step of the process of the invention the alkylation may be effected in the presence of a tertiary amine, preferably triethylamine or tetramethylethylenediamine. The same reaction zone or vessel as for the hydrolysis, and the same solvents as described for the first step may be used. The alkylation may be effected under mild conditions, e.g. at 0° to 100°C, preferably at 80°C, using 3'-phenoxybenzyl chloride, 3'-phenoxybenzyl bromide or 3'-phenoxybenzyl iodide as the alkylating agent. When the hydrolysis in the first step is carried out with an alkali metal hydroxide or alkaline earth metal hydroxide, the alkylation reaction in the second step may be carried out in presence of a catalyst, e.g. a tertiary amine, preferably triethylamine or tetramethylethylene diamine, as already mentioned.

The compound of formula I may be converted into a pyrethroid of formula II by oxidation with chromium trioxide in pyridine to yield the corresponding aldehyde ester, followed by subjection of the said aldehyde ester to a Wittig reaction with triphenylphosphine or hexamethylphosphotriamide in carbon tetrachloride to give the product of formula II.

The invention will now be further illustrated by way of the following Examples, which demonstrate processes for preparing (1R,Cis)-5-phenoxybenzyl-2,2-dimethyl-3-hydroxymethyl cyclopropane carboxylate.

The identity and purity of the product in each Example was determined by spectroscopic and GLC analysis.

## EXAMPLE 1

To a stirred solution comprising 12.6 g (0.1 mol) of the γ-lactone of (-)-(1R, Cis)-dimethyl-3'-hydroxymethyl cyclopropane-1-carboxylic acid of formula (IV) herein in 30 ml of ethanol were added 80 ml of a 5% aqueous sodium hydroxide solution. The reaction mixture thus prepared was stirred for 1 hour at ambient temperature after which the solvent was removed to yield the sodium salt of the (1R, Cis)-acid of formula (IV) as a pale yellow solid.

The salt obtained was dissolved in 40 ml ethanol to which 26.3 g (0.1 mol) 3'-phenoxybenzyl bromide in 20 ml ethanol were added. This reaction mixture was refluxed for 2 hours after addition of 1.0 g (0.01 mol) of triethylamine. Ethanol was removed and the residue extracted with dichloromethane (2 x 100 ml). The organic layer was washed with saturated brine solution and dried. Removal of the solvent yielded (-)-(1R, Cis)-3'-phenoxybenzyl 2,2-dimethyl-3-hydroxymethyl cyclopropane carboxylate of the formula (I) herein, (30.5 g, 93.5%). The product obtained was then purified by column chromatography over silica gel using as eluent a mixture of n-hexane and chloroform in a ratio of 9:1 to yield the pure ester of formula (I) as a yellow viscous oil (25 g, 80%) $[\alpha]_D^{25}$ -5.0°(C 2, $CHCl_3$).

EXAMPLE 2

The same procedure was followed as in Example 1 except that for the hydrolysis, 12.6 g (0.01 mol) of the γ-lactone of (-)-(1R, Cis)-dimethyl-3'-hydroxymethyl cyclopropane-1-carboxylic acid of formula (IV) in 30 ml of ethanol were employed with a 5% aqueous sodium hydroxide solution. The only other difference from Example I was that the subsequent alkylation of the hydrolysate with 3'-phenoxybenzyl bromide was carried out in the presence of 1.16 g (0.01 mol) tetramethylethylene diamine as catalyst.

The final product comprised the pure ester of formula (I) the yield being 25.5 g, 82% $[\alpha]D^{25}$ - 5.0° (C 2, $CHCl_3$).

EXAMPLE 3

The same procedure was followed as in Example 1 except that for the hydrolysis, 6.3 g (0.05 mol) of the γ-lactone of (-)-(1R, Cis)-dimethyl-3'-hydroxymethyl cyclopropane-1-carboxylic acid of formula (IV) in 20 ml of 1-propanol or 2-propanol were added to 40 ml of a 5% aqueous sodium hydroxide solution while the subsequent alkylation of the hydrolysate was effected with 13.20 g (0.05 mol) 3'-phenoxybenzyl bromide in 10 ml of 1-propanol or 2-propanol.

0161827

The final product comprised the pure ester of formula (I) in the form of a yellow viscous oil, the yield being 12.1 g, 78% [α]D$^{25}$ - 4.98° (C 1, $CHCl_3$).

EXAMPLE 4

The procedure of Example 1 was repeated employing for the hydrolysis 6.3 g (0.05 mol) of the γ-lactone of (-)-(1R, Cis)-dimethyl-3'-hydroxymethyl cyclopropane-1-carboxylic acid of formula (IV) in 20 ml of ethanol and 130 ml of a 10% ethanolic solution of tetra n butyl ammonium hydroxide. Hydrolysis was effected for 2 hours at ambient temperature followed by alkylation of the hydrolysate with 26.3 g (0.01 mol) 3'-phenoxybenzyl bromide. No amine catalyst was present during alkylation.

After purification, the final product comprised the pure ester of formula (I), the yield being 21.2 g, 65% [α]D$^{25}$ - 5.05° (C 1, $CHCl_3$).

EXAMPLE 5

The procedure of Example 1 was followed. For the hydrolysis there was employed 2.52 g (0.02 mol) of the γ-lactone of (-)-(1R, Cis)-dimethyl-3'-hydroxymethyl cyclopropane-1-carboxylic acid of formula (IV) in 10 ml of ethanol or 1-propanol or 2-propanol with 16 ml of a 5%

aqueous sodium hydroxide solution. The hydrolysate was subjected to alkylation by means of 4.4 g (0.02 mol) 3'-phenoxybenzyl chloride in the presence of 0.2 g triethylamine or 0.23 g tetramethylethylene diamine as catalyst.

After purification, the final product comprised the pure ester of formula (I), the yield being 3.7 g, 60% $[\alpha]D^{25}$ - 5.0° (C 1, $CHCl_3$).

EXAMPLE 6

Procedure identical to that of Example (V) was followed with the exception that for alkylation of the hydrolysate, 6.2 g (0.02 mol) 3'-phenoxybenzyl iodide was employed instead of the chloride. Other reactants and amounts were maintained.

The final product comprised the pure ester of formula (I), the yield being 5.26 g, 81% $[\alpha]D^{25}$ - 5.08° (C 1, $CHCl_3$).

CLAIMS

1. (1R, Cis)-3'-phenoxybenzyl-2,2-dimethyl-3-hydroxymethyl cyclopropane carboxylate of formula:

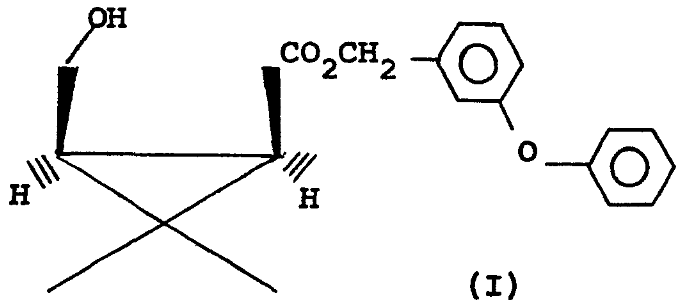

(I)

2. A process for the preparation of (1R, Cis)-3'-phenoxybenzyl-2,2-dimethyl-3-hydroxymethyl cyclopropane carboxylate

which comprises, in a first step, hydrolysing the γ-lactone of (1R, Cis)-2,2-dimethyl-3-hydroxymethyl cyclopropane-1-carboxylic acid of formula:

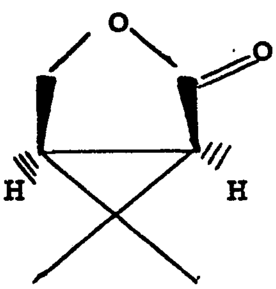

(IV)

and, in a second step, reacting the intermediate thus formed with a 3'-phenoxybenzyl halide having of formula:

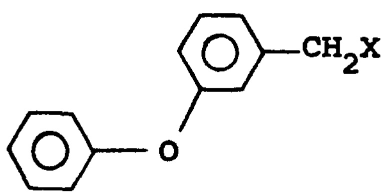

(V)

wherein X represents a halide.

3. A process according to claim 2 wherein the hydrolysis is carried out with a base in the presence of a solvent.

4. A process according to claim 3 wherein the base is an alkali metal hydroxide, an alkaline earth metal hydroxide or a quaternary ammonium hydroxide, or a mixture thereof.

5. A process according to claim 3 or 4 wherein the solvent is an alkanol having from 1 to 4 carbon atoms, dichloromethane or tetrahydrofuran, or a mixture thereof.

6. A process according to any one of claims 2 to 5 wherein the hydrolysis is carried out at a temperature of from 0° to 100°C.

7. A process according to any one of claims 2 to 6 wherein the 3'-phenoxybenzyl halide is 3'-phenoxybenzyl chloride, 3'-phenoxybenzyl bromide or 3'-phenoxybenzyl iodide.

8. A process according to any one of claims 2 to 7 wherein the reaction of the intermediate with the 3'-phenoxybenzyl halide is carried out in the presence of a catalyst.

9. A process according to claim 8 wherein the catalyst is triethylamine or tetramethylethylene diamine, or a mixture thereof.

10. A process according to any one of claims 2 to 9 wherein the reaction of the intermediate with the 3'-phenoxybenzyl halide is carried out at a temperature of from 0° to 100°C.

0161827

European Patent Office

# EUROPEAN SEARCH REPORT

Application number

EP 85 30 2767

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 100, no. 7, 13th February 1984, page 543, no. 51146t, Columbus, Ohio, US; JP - A - 58 146 542 (SAGAMI CHEM. RES. CENTER) 01-09-1983 ---- | 2-10 | C 07 C 69/757<br>C 07 C 67/02 |
| Y | JOURNAL OF THE AMERICAN CHEMICAL SOCIETY, vol. 104, no. 17, 25th August 1982, pages 4659-4665, US; I.J. JAKOVAC et al.: "Enzymes in organic synthesis. 24.1 Preparations of enantiomerically pure chiral lactones via stereospecific horse liver alcohol dehydrogenase catalyzed oxidations of monocyclic meso diols2" ----- | 2-10 | |
| | | | TECHNICAL FIELDS SEARCHED (Int. Cl.4) |
| | | | C 07 C 69/00 |

The present search report has been drawn up for all claims

| Place of search | Date of completion of the search | Examiner |
|---|---|---|
| THE HAGUE | 25-07-1985 | WRIGHT M.W. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82